# EUROPEAN PATENT SPECIFICATION

(11) **EP 0 930 047 B1**
(45) Date of publication and mention of the grant of the patent: **09.01.2008**
(21) Application number: 99610012.9
(22) Date of filing: 18.01.1999
(51) Int. Cl.: A61B 17/00

(54) **Specimen retrieval pouch**
Probenentnahmebeutel& x9;
Poche de récupération d'échantillons

(30) Priority: 19.01.1998 DK 6698
(43) Date of publication of application: 21.07.1999
(73) Proprietor: Lina Medical ApS, 2600 Glostrup (DK)
(72) Inventor: Kornerup, Niels, 2960 Rungsted Kyst (DK)
(74) Representative: Nielsen, Henrik Sten

(56) References cited:
- WO-A-93/15671
- WO-A-93/17630
- US-A- 5 312 416
- US-A- 5 480 404

## Description

The present invention relates to a means for removing unwanted particles such as damaged tissues from the ventral cavity of a patient, and more specifically to a retractable endoscopic bag with adjustable opening.

From US 5,480,404, a retractable endoscopic instrument is known, which instrument comprises an endoscopic bag. The endoscopic instrument further comprises a retractable rod defining a distal end and a proximal end, an intermediate rod having an axially extending through-going passage for receiving the retractable rod allowing the retractable rods to slide inside the intermediate rod and further a drawstring connected to the retractable rod at the distal end thereof. The endoscopic bag is joined to the drawstring allowing the opening of the endoscopic bag defined by the drawstring to be closed off by pulling the drawstring into the retractable rod by means of the intermediate rod. The instrument also has a connector at the distal end of the intermediate rod, which connector allows the instrument in its entity to be joined to a similar connector of a trocar having a trocar tube, through which the endoscopic instrument is to be introduced into the body cavity of a patient. By the introduction of the instrument into the trocar and the trocar tube as the endobag is freely exposed at the outer end of the retractable rod prior to the introduction of the endobag and the retractable rod into the trocar and the trocar tube. Furthermore, the retractable endoscopic instrument known from US 5,480,404 fails to include the feature characteristic of the present invention as defined in the characterising part of independent claim 1.

From published international patent application, application No. PCT/US93/01977, publication No. WO 93/17630, a retractable endoscopic instrument of the pre-characterising part of independent claim 1 is known. Like the retractable endoscopic instrument known from the above US patent, the retractable endoscopic instrument known from the above published international patent application fails to describe the feature characteristic of the present invention as defined in the characterising part of independent claim 1.

From DK Utility Model No. 00126 U3 which is assigned to the assignee of the present invention, an endoscopic surgical bag of the pre-characterising part of independent claim 1 is known.

DK Utility Model No. 00126 U3 discloses a bag for use in endoscopic surgical operations which bag may open and close easily and gradually by means of a slidable rod. Furthermore, it is not necessary to move the bag itself in order to open or close it. The device disclosed in DK Utility Model No. 00126 U3 consists of a retractable rod which slides inside a trocar, a control rod which is used for directing the bag forward and backward and laterally, a drawstring attached with one end to the control rod and with the other end to the retractable rod, and a bag welded to the drawstring. While the above-mentioned device provides the advantages over prior art devices that the bag may be opened and closed gradually several times and that the bag may be opened without being necessary to move the bag itself and in particular the bag opening, the spot welding of the bag directly to the drawstring has the drawback that the surface of attachment between the bag and the drawstring is limited to a thin contact line between said bag and said drawstring, which poses the danger that, due to the weight of the contents of the bag and the form of attachment between said bag and said drawstring, the bag may disconnect itself from the drawstring partially or totally and remain squeezed inside the ventral cavity of the patient, prolonging the operation time and affecting negatively the operation itself, sometimes with the risk of endangering the patient's life.

A disadvantage of the invention according to the prior art is the connection between the drawstring and the retractable rod by means of an adhesive, which connection has proved unsafe in use.

Accordingly, it is an object of the present invention to provide an improved endoscopic surgical bag which eliminates the drawback and disadvantage of the prior art endoscopic surgical bag.

Another object of the present invention is to provide an endoscopic surgical bag of high mechanical strength and integrity as compared to the prior art endoscopic surgical bag.

These objects of the present invention are achieved by means of a retractable endoscopic instrument for use during endoscopic surgery for receiving and encapsulating bulky material produced during said endoscopic surgery, comprising: a retractable rod defining a distal end and a proximal end; an intermediary rod having an axially extending through-going passage for receiving said retractable rod allowing said retractable rod to slide inside said intermediary rod; a protection sheath circumferentially encircling and receiving said intermediary rod and allowing said retractable rod and said intermediary rod to slide inside said protection sheath; a drawstring connected to said retractable rod at said distal end thereof; and an endoscopic bag having an outer edge defining an opening for receiving said bulky material and being connected to said drawstring so as to cause said opening to be closed off by said drawstring tightening along said outer edge; characterized in that said drawstring is connected to said retractable rod by means of a pair of cooperating and mating parts of said drawstring and retractable rod constituting an input part and a receiving part, and that said margin of said endoscopic bag is folded back over said drawstring and attached onto itself, forming a passage that allows said drawstring to freely slide therethrough and cause said opening to be closed or opened.

The present invention provides thus an improved attachment of the drawstring to the retractable rod, the new attachment form being achieved by means of a junction between a particularly shaped input end of the drawstring and a matching rereceiving end of the retractable rod, the result being a functionally more reliable bag. The attachment of the endoscopic bag onto itself is achieved by means of any of a number of well-known means of attachment such as vulcanization or lamination, bonding by means of an adhesive or stitching by means of a thread-like material.

In one embodiment one end of the drawstring is fastened to a fixed U-shaped guide solidary with an intermediary rod while the other end, being connected to a retractable rod traversing the intermediary rod, slides through the guide so that the bag opening may close gradually.

In another embodiment one end of the drawstring is fastened to a portion of the same drawstring so that the drawstring ends may upon pulling of the retractable rod be retracted inside the intermediary rod simultaneously and the bag opening may close gradually at double the closing speed of the first embodiment.

The endoscopic bag must be made of a moisture-proof material which must prevent the transmission of fluid or malignant tissue cells to healthy tissue within the surgical site and also be puncture-resistant so as to resist penetration or damage by a surgical instrument, such a suitable bag material being HDPE.

The drawstring, having to display sufficient flexibility, may advantageously be made of nylon.

The retractable rod which is attached to the drawstring in order to allow the sliding thereof through the intermediary rod must be sufficiently rigid and may be made of a plastic material showing sufficient rigidity such as polyvinylchloride.

The protection sheath which can contain the entire endoscopic bag when the bag is in its most retracted position has an outer diameter of approx. 10 mm and may advantageously be made of a transparent or at least radio-transparent plastic material such as polycarbonate or polyvinylchloride.

The invention will now be further described with reference to the drawings, in which:
Fig. 1a is a perspective view of the retractable endoscopic bag according to the present invention, seen in a position fully retracted inside the protection sheath,
Fig. 1b is a perspective view of the endoscopic bag according to the present invention, seen in a partially open position outside the protection sheath,
Fig. 1c is a perspective view of the endoscopic bag according to the present invention, seen in a fully open position outside the protection sheath,
Fig. 2 shows in a partially perspective, partially cross-sectional view the extraction of damaged tissue or other unwanted particles from the ventral cavity of a patient,
Fig. 3a shows in a detailed perspective view the separate joining parts constituting the connection between the retractable rod and the bag-supporting drawstring,
Fig. 3b shows in a detailed perspective view the connected joining parts constituting the connection between the retractable rod and the bag-supporting drawstring,
Fig. 4a is a detailed perspective view of a first embodiment of the drawstring attachment system with one mobile end, and
Fig. 4b is a detailed perspective view of a second embodiment of the drawstring attachment system with simultaneously mobile ends.

In the following examples identical component parts of the various embodiments are designated identical reference numerals.

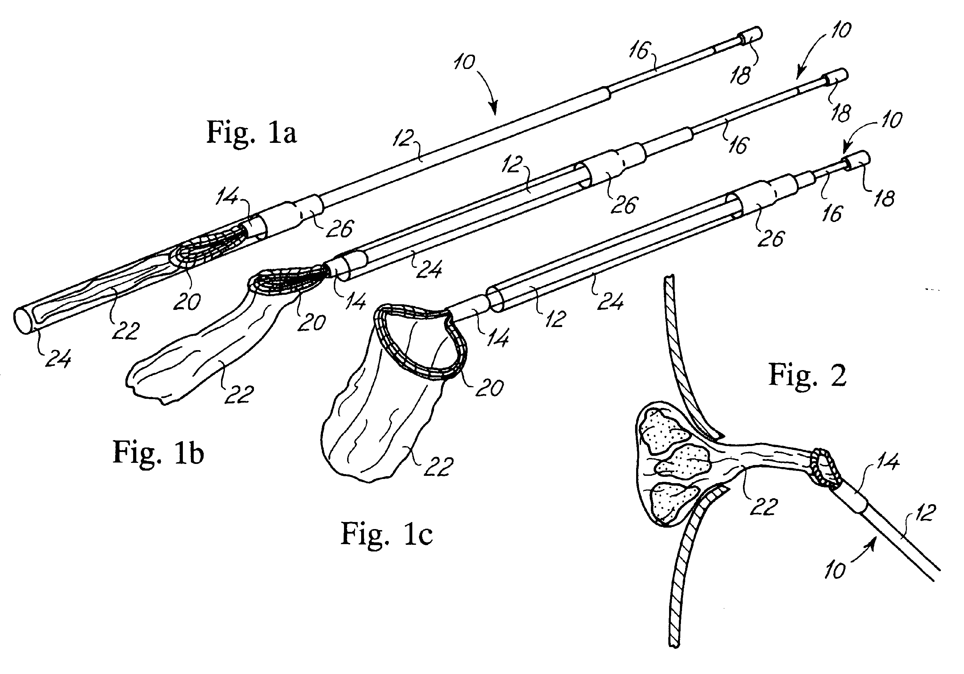

In Figs. 1a, 1b and 1c the retractable endoscopic bag according to the present invention is designated the reference numeral 10 in its entirety.

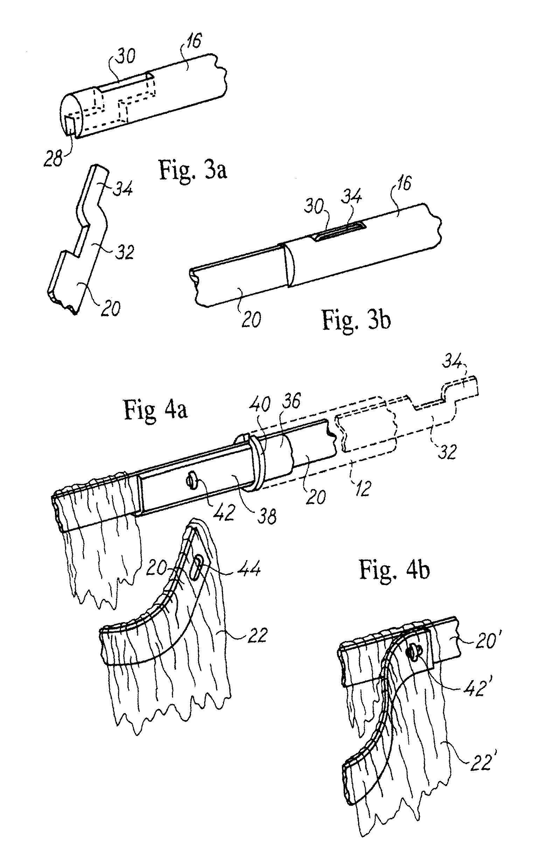

Fig. 1 shows in a perspective view a retractable endoscopic bag 10 according to the present invention, seen in a position fully retracted inside a protection sheath 24. The protection sheath 24 has its one end free and the other end surrounded by a sealing sleeve 26. Through the protection sheath 24 slides a cylindrical intermediary rod 12, which is traversed by a retractable rod 16 connected at its end corresponding to the free end of the protection sheath 24 to a drawstring 20 having a first end and a second end and around which an endoscopic bag 22 is attached by having the margin thereof folded back over the drawstring 20 and welded to the body of the same endoscopic bag 22. More detailed views of the connection between the retractable rod 16 and the bag-supporting drawstring 20 are shown in Figs. 3a and 3b. Fig. 3a shows in a detailed perspective view the separate joining parts constituting the connection between the retractable rod 16 and the bag-supporting drawstring 20, the first end of the bag-supporting drawstring 20 being shaped such as to fit into matching spaces provided inside the end of the retractable rod 16. More precisely, the drawstring 20 is provided with an intermediary cutout that transforms the first end of the drawstring 20 into a joining part 32 and with a final cutout carried out on the part of the drawstring opposite the first cutout so that the drawstring ends in a joining part extension 34. These two joining parts 32 and 34 fit into matching cutouts 28 and 30, respectively, provided in the retractable rod 16, allowing forced movement of the drawstring 20 by forward and backward movement of the retractable rod 16. The joined assembly consisting of the drawstring 20 with its joining parts 32 and 34 fitted into the retractable rod 16 is shown in Fig. 3b, where the extension 34 is seen to match the cutout 30. The second end of the drawstring 20 may be attached to either a fixed or a mobile part of the retractable bag 10, defining thus two embodiments of the retractable bag 10, as depicted in Figs. 4a and 4b.

Fig. 4a is a detailed perspective view of a first and presently preferred embodiment of the bag attachment system, according to which the first end of the drawstring 20 slides through a U-shaped guide 38 solidary with a stop collar 36 fitted into the intermediary rod 12. The U-shaped guide 38 is provided on its lateral side corresponding to the interior of the bag with a vertical, oblong knob 42 having a base of a height corresponding to the drawstring thickness, which knob 42 matches an oblong hole 44 provided in the second end of the drawstring 20 containing the endoscopic bag 22. The second end of the flexible drawstring 20 is superposed the vertical knob 42, pressed thereon and then rotated 90° so that the second end is secured to the U-shaped guide 38. The attachment of the second end of the drawstring 20 to the fixed U-shaped guide 38 provides, when pulling the retractable rod 16, the gradual narrowing of the opening of the retractable endoscopic bag 22 and the gradual insertion of the endoscopic bag 22 into the intermediary rod 12.

A different attachment solution for the second end of the drawstring 20 is shown in Fig. 4b, in which the knob 42' is provided on the drawstring 20', so that the pulling movement of the retractable rod 16 solidary with the drawstring 20 retracts both drawstring ends into the intermediary rod 12, providing a retraction speed of the drawstring that is double the retraction speed of the drawstring in the first embodiment.

Fig. 1b is a perspective view of the endoscopic bag 10 according to the present invention, seen in a partially open position outside the protection sheath 24. Pushing of the retractable rod 16 into the protection sheath and into the ventral cavity of the patient is facilitated by the provision at the manoeuvering end of the retractable rod 16 of a handle 18.

Fig. 1c is a perspective view of the endoscopic bag 10 according to the present invention, seen in a fully open position outside the protection sheath 24.

Fig. 2 shows in a partially perspective, partially cross-sectional view the extraction of damaged tissue or other unwanted particles from the ventral cavity of a patient. To extract unwanted particles from the ventral cavity of a patient the endoscopic bag 10 is introduced through a surgical passageway in the ventral cavity of the patient with the drawstring 20 and the endoscopic bag 22 fully retracted inside the protection sheath 24 so that the movement of the bag assembly is not impeded by an eventual grip of the drawstring 20 or of the endoscopic bag 22 in the internal organs or other body parts of the patient. Once the endoscopic bag 10 has reached the zone in which the unwanted particles are found, the retractable rod 16 solidary with the drawstring 20 is pushed by means of the handle 18 through the protection sheath 24 so that the drawstring 20 slides out of the intermediary rod 12 opening the endoscopic bag 22. The particles to be removed from the patient's ventral cavity are then caught inside the bag 22, the retractable rod 16 is partially retracted inside the intermediary rod 12 closing the bag 22 partially, according to the size of the particles to be removed, and the endoscopic bag 22 containing the particles is pulled through the surgical passageway.

A prototype implementation of the retractable bag according to the present invention was made from the following components:
The retractable rod (16) had a length of 40 cm and a diameter of 4 mm and was made from polycarbonate, the intermediary rod (12) had also a length of 40 cm and a diameter of 4 mm but was made from polyvinylchloride, the protection sheath (24) had an inner diameter of 5 mm and a length of 25 cm and was made from polycarbonate, and the drawstring (20) had a the length of 30 cm and was made from HDPE.

## Claims

1. A retractable endoscopic instrument (10) for use during endoscopic surgery for receiving and encapsulating bulky material produced during said endoscopic surgery, comprising:
a retractable rod (16) defining a distal end and a proximal end;
an intermediary rod (12) having an axially extending through-going passage for receiving said retractable rod (16) allowing said retractable rod (16) to slide inside said intermediary rod (12);
a protection sheath (24) circumferentially encircling and receiving said intermediary rod (12) and allowing said retractable rod (16) and said intermediary rod (12) to slide inside said protection sheath (24);
a drawstring (20) connected to said retractable rod (16) at said distal end thereof; and
an endoscopic bag (22) having an outer margin defining an opening for receiving said bulky material and being connected to said drawstring (20) so as to cause said opening to be closed off by said drawstring (20) tightening along said outer edge; **CHARACTERIZED in that** said drawstring (20) is connected to said retractable rod (16) by means of a pair of cooperating and mating parts (32, 34; 28,30) of said drawstring (20) and retractable rod (16) constituting an input part (32, 34) and a receiving part (28, 30), and that said margin of said endoscopic bag (22) is folded back over said drawstring (20) and attached onto itself, forming a passage that allows said drawstring (20) to freely slide therethrough and cause said opening to be closed or opened.

2. Retractable endoscopic instrument (10) according to claim 1, **CHARACTERIZED in that** the attachment of the margin of said endoscopic bag (22) onto itself is realized by welding.

3. Retractable endoscopic instrument (10) according to claim 1, **CHARACTERIZED in that** the attachment of the margin of said endoscopic bag (22) onto itself is realized by bonding by means of an adhesive.

4. Retractable endoscopic instrument (10) according to claim 1,**CHARACTERIZED in that** the attachment of the margin of said endoscopic bag (22) onto itself is realized by stitching by means of a thread-like material.

5. Retractable endoscopic instrument (10) according to any one of the claims 1-4, **CHARACTERIZED in that** one end of said drawstring (20) is attached to a fixed U-shaped guide (38) solidary with said intermediary rod (12) while the other end is capable to slide through said guide (38), allowing said bag (22) opening to close gradually.

6. Retractable endoscopic instrument (10) according to any one of the claims 1-4, **CHARACTERIZED in that** one end of said drawstring (20) is attached to a portion of the same drawstring (20), allowing said bag (22) opening to close gradually at a speed which is double the closing speed of the bag (22) according to claim 5.

7. Retractable endoscopic instrument (10) according to any of the preceding claims, **CHARACTERIZED in that** said receiving part (28, 30) has the shape of two partially superposed parallelepipeds and said mating input part (32, 34) comprises a first cutout on one side of the connecting end of said drawstring (20) and a second cutout on the opposite side of said drawstring (20), said first and second cutout being staggered.

8. Retractable endoscopic instrument (10) according to any one of the claims 1-7, **CHARACTERIZED in that** said input part (32, 34) is shaped on said drawstring (20) and said mating receiving part (28, 30) is shaped on said retractable rod (16).

9. Retractable endoscopic instrument (10) according to any one of the claims 1-7, **CHARACTERIZED in that** said input part (32, 34) is shaped on said retractable rod (16) and said mating receiving part (28, 30) is shaped on said drawstring (20).

10. Retractable endoscopic instrument (10) according to any of the preceding claims, **CHARACTERIZED in that** said endoscopic bag (22) is made from a moisture-proof, puncture-resistant, transparent or at least radio-transparent plastic material such as polycarbonate or polyvinylchloride.

## Patentansprüche

1. Einschiebbare Endoskopvorrichtung (10) zur Verwendung im Rahmen eines als Endoskopie durchgeführten chirurgischen Eingriffes, welches während des besagten als Endoskopie durchgeführten chirurgischen Eingriffes entferntes sperriges Material umschließen und aufnehmen kann, umfassend:
ein einschiebbares starres Führungselement (16), welches ein fernes und ein nahes Ende aufweist;
ein dazwischen geschaltetes starres Führungselement (12), welches einen Durchgang besitzt, der sich axial erstreckt um das einschiebbare starre Führungselement (16) aufzunehmen und erlaubt, das besagte einschiebbare starre Führungselement (16) in das dazwischen geschaltete starre Führungselement (12) einzuschieben;
eine Schutzhülle (24), welche das besagte dazwischen geschaltete starre Führungselement (12) aufnehmen und ringsum umschließen kann und es erlaubt, das besagte einschiebbare starre Führungselement (16) und das besagte dazwischen geschaltete starre Führungselement (12) in besagte Schutzhülle (24) einzuschieben;
einen Kordelzug (20) welcher mit dem besagten einschiebbaren starren Führungselement (16) an dessen fernem Ende verbunden ist;
einen Endoskopbeutel (22), welcher eine äußere Begrenzung mit einer Öffnung aufweist, um besagtes entferntes sperriges Material aufnehmen zu können und mit dem Kordelzug (20) auf solche Weise verbunden ist, dass bei Zug an der Kordel (20) sich der Beutel an besagter äußerer Begrenzung schließt;
**dadurch gekennzeichnet, dass** der besagte Kordelzug (20) mit besagtem einschiebbarem starrem Führungselement (16) mit Hilfe eines Paares von zusammenarbeitenden und gegenseitig passenden männlichen und weiblichen Teilen (32, 34; 28, 30) des besagten Kordelzuges (20) und einschiebbaren starren Führungselements (16), welche auf diese Weise einen Zugangs- (32, 34) und einen Aufnahmeteil (28, 30) ausbilden und dass besagte Begrenzung des Endoskopbeutels (22) über den besagten Kordelzug (20) zurückgefaltet und an diesem durch eine feste Verbindung mit sich selbst so befestigt ist, dass sich ein Durchgang ergibt, der die freie Bewegung des besagten Kordelzuges (20) durch den Durchgang erlaubt und das Öffnen und Schließen der Öffnung bewirkt.

2. Einschiebbare Endoskopvorrichtung (10) gemäß Anspruch 1, **dadurch gekennzeichnet, dass** die Verbindung der Begrenzung des Endoskopbeutels (22) mit sich selbst durch Schweißen verwirklicht ist.

3. Einschiebbare Endoskopvorrichtung (10) gemäß Anspruch 1, **dadurch gekennzeichnet, dass** die Verbindung der Begrenzung des Endoskopbeutels (22) mit sich selbst durch Verkleben mittels eines Klebers verwirklicht ist.

4. Einschiebbare Endoskopvorrichtung (10) gemäß Anspruch 1, **dadurch gekennzeichnet, dass** die Verbindung der Begrenzung des Endoskopbeutels (22) mit sich selbst durch Vernähen mit einem Fadenähnlichen Material verwirklicht ist.

5. Einschiebbare Endoskopvorrichtung (10) gemäß einem der Ansprüche 1 bis 4, **dadurch gekennzeichnet, dass** das eine Ende des besagten Kordelzuges (20) mit einer festen U-förmigen Führungsschiene (38) versehen welche mit dem besagten dazwischen geschalteten starren Führungselement (12) stabil verbunden ist, während das andere Ende in besagter Führungsschiene (38) gleiten kann, wodurch die Öffnung des besagten Beutels (22) schrittweise geschlossen werden kann.

6. Einschiebbare Endoskopvorrichtung (10) gemäß einem der Ansprüche 1 bis 4, **dadurch gekennzeichnet, dass** das eine Ende des besagten Kordelzuges (20) an einer solchen Stelle des selben Kordelzuges (20) befestigt ist, dass die Öffnung des besagten Beutels (22) schrittweise mit einer im Vergleich zu dem Beutel gemäß Anspruch 5 doppelten Geschwindigkeit geschlossen werden kann.

7. Einschiebbare Endoskopvorrichtung (10) gemäß einem der vorangehenden Ansprüche, **dadurch gekennzeichnet, dass** das besagte weibliche Teil (28, 30) die Form von zwei teilweise überlappenden Parallelepipedonen aufweist und das passende männliche Teil (32, 34) einen ersten Einschnitt auf der dem verbindenden Ende des besagten Kordelzuges (20) zugewandten Seite umfasst sowie einen zweiten Einschnitt auf der dem besagten Kordelzuges (20) abgewandten Seite aufweist, wobei der erste und der zweite Einschnitt versetzt angeordnet sind.

8. Einschiebbare Endoskopvorrichtung (10) gemäß einem der Ansprüche 1 bis 7, **dadurch gekennzeichnet, dass** das besagte passende männliche Teil (32, 34) aus dem besagten Kordelzug (20) ausgeformt ist und das besagte passende weibliche Teil (28, 30) aus dem besagten einschiebbaren starren Führungselement (16) ausgeformt ist.

9. Einschiebbare Endoskopvorrichtung (10) gemäß einem der Ansprüche 1 bis 7, **dadurch gekennzeichnet, dass** das besagte passende männliche Teil (32, 34) aus dem besagten einschiebbaren starren Führungselement (16) ausgeformt ist und das besagte passende weibliche Teil (28, 30) aus dem besagten Kordelzug (20) ausgeformt ist.

10. Einschiebbare Endoskopvorrichtung (10) gemäß einem der vorangehenden Ansprüche, **dadurch gekennzeichnet, dass** der besagte Endoskopbeutel (22) aus wasserfestem, durchstoßfestem, transparentem oder wenigstens radio-transparentem Plastikmaterial wie zum Beispiel Polycarbonat oder Polyvinylchlorid hergestellt ist.

## Revendications

1. Instrument endoscopique rétractable (10) destiné à une utilisation en chirurgie endoscopique pour recevoir et encapsuler un matériau volumineux produit pendant ladite chirurgie endoscopique, comprenant :
une tige rétractable (16) définissant une extrémité distale et une extrémité proximale;
une tige intermédiaire (12) présentant un passage traversant s'étendant axialement, destiné à recevoir ladite tige rétractable (16) et permettant à ladite tige rétractable (16) de coulisser à l'intérieur de ladite tige intermédiaire (12) ;
une gaine de protection (24) encerclant circonférentiellement et recevant ladite tige intermédiaire (12) et permettant à ladite tige rétractable (16) et à ladite tige intermédiaire (12) de coulisser à l'intérieur de ladite gaine de protection (24) ;
un cordon de serrage (20) relié à ladite tige rétractable (16) au niveau de ladite extrémité distale de celle-ci; et
un sac endoscopique (22) ayant un bord extérieur définissant une ouverture pour recevoir ledit matériau volumineux et étant relié audit cordon de serrage (20) afin de provoquer la fermeture de l'ouverture par le serrage dudit cordon de serrage (20) le long dudit bord extérieur ;
***caractérisé en ce que*** ledit cordon de serrage (20) est relié à ladite tige rétractable (16) au moyen d'une paire de parties coopérant et se conjuguant (32, 34 ; 28, 30) desdits cordon de serrage (20) et tige rétractable (16) constituant une partie d'entrée (32, 34) et une partie de réception (28, 30), et ***en ce que*** ledit bord dudit sac endoscopique (22) est replié sur ledit cordon de serrage (20) et fixé à lui-même en formant un passage qui permet audit cordon de serrage (20) de coulisser librement dans ce passage et permet de fermer ou d'ouvrir ladite ouverture.

2. Instrument endoscopique rétractable (10) selon la revendication 1, ***caractérisé en ce que*** la fixation du bord dudit sac endoscopique (22) sur lui-même est réalisée par soudage.

3. Instrument endoscopique rétractable (10) selon la revendication 1, ***caractérisé en ce que*** la fixation du bord dudit sac endoscopique (22) sur lui-même est réalisée par collage au moyen d'un adhésif.

4. Instrument endoscopique rétractable (10) selon la revendication 1, ***caractérisé en ce que*** la fixation du bord dudit sac endoscopique (22) sur lui-même est réalisée par couture au moyen d'un matériau de type fil.

5. Instrument endoscopique rétractable (10) selon l'une quelconque des revendications 1 à 4, ***caractérisé en ce qu*'**une première extrémité dudit cordon de serrage (20) est fixée à un guide fixe (38) en forme de U solidaire de ladite tige intermédiaire (12) alors que l'autre extrémité est apte à coulisser à travers ledit guide (38), permettant à l'ouverture dudit sac (22) de se refermer graduellement.

6. Instrument endoscopique rétractable (10) selon l'une quelconque des revendications 1 à 4, ***caractérisé en ce qu'***une première extrémité dudit cordon de serrage (20) est fixée à une portion du même cordon de serrage (20), permettant à l'ouverture dudit sac (22) de se refermer progressivement à une vitesse qui est double de la vitesse de fermeture du sac (22) selon la revendication 5.

7. Instrument endoscopique rétractable (10) selon l'une quelconque des revendications précédentes, ***caractérisé en ce que*** ladite partie de réception (28, 30) a la forme de deux parallélépipèdes partiellement superposés et ladite partie d'entrée (32, 34) se conjuguant comprend une première découpe sur un côté de l'extrémité de connexion dudit cordon de serrage (20) et une deuxième découpe sur le côté opposé dudit cordon de serrage (20), lesdites première et deuxième découpes étant décalées.

8. Instrument endoscopique rétractable (10) selon l'une quelconque des revendications 1 à 7, ***caractérisé en ce que*** ladite partie d'entrée (32, 34) est façonnée sur ledit cordon de serrage (20) et ladite partie de réception se conjuguant (28, 30) est façonnée sur ladite tige rétractable (16).

9. Instrument endoscopique rétractable (10) selon l'une quelconque des revendications 1 à 7, ***caractérisé en ce que*** ladite partie d'entrée (32, 34) est façonnée sur ladite tige rétractable (16) et ladite partie de réception se conjuguant (28, 30) est façonnée sur ledit cordon de serrage (20).

10. Instrument endoscopique rétractable (10) selon l'une quelconque des revendications précédentes, ***caractérisé en ce que*** ledit sac endoscopique (22) est réalisé dans une matière plastique à l'épreuve de l'humidité, anti-perforation, transparente ou tout au moins transparente aux rayons X, telle que du polycarbonate ou du polychlorure du vinyle.
